# EUROPEAN PATENT SPECIFICATION

(11) **EP 1 879 869 B1**
(45) Date of publication and mention of the grant of the patent: **14.01.2009**
(21) Application number: 06742664.3
(22) Date of filing: 25.04.2006
(51) Int. Cl.: C07D 231/44

(54) **PROCESS FOR THE PREPARATION OF PRECURSORS OF 5-ALKYLTHIOALKYLAMINO-1-PHENYL-PYRAZOLES**
VERFAHREN ZUR HERSTELLUNG VON VORSTUFEN FÜR 5-ALKYLTHIOALKYLAMINO-1-PHENYL-PYRAZOLE
PROCEDE DE PREPARATION DE PRECURSEURS DE 5-ALKYLTHIOALKYLAMINO-1-PHENYL-PYRAZOLES

(30) Priority: 07.05.2005 EP 05009998
(43) Date of publication of application: 23.01.2008
(73) Proprietor: Merial Limited, Duluth, GA 30096-4640 (US)
(72) Inventor: SCHNATTERER, Stefan, 65795 Hattersheim (DE); MAIER, Michael, 65931 Frankfurt (DE); LOCHHAAS, Friederike, 36341 Lauterbach (DE); KNAUF, Werner, 76887 Bad Bergzabern (DE); SEEGER, Karl, 65719 Hofheim (DE)
(74) Representative: Clyde-Watson, Zöe
(86) International application number: PCT/EP2006/003771
(87) International publication number: WO 2006/119862

(56) References cited:
- EP-A- 0 295 117
- WO-A-20/05023775
- DE-A1- 3 719 732
- DATABASE BEILSTEIN Beilstein Institut zur Foerderung der Wissenschaften, Farnkfurt/Main, DE; XP002357309 & J. CHEM. SOC. CHEM. COMMUN., vol. 9, 1994, pages 1083-1084,
- DATABASE BEILSTEIN Beilstein Institut zur Foerderung der Wissenschaften, Farnkfurt/Main, DE; XP002357310 & J. CHEM. SOC. CHEM. COMMUN., 1989, pages 518-520,
- DATABASE BEILSTEIN Beilstein Institut zur Foerderung der Wissenschaften, Farnkfurt/Main, DE; XP002357311 & J. MED. CHEM., vol. 38, no. 12, 1995, pages 2217-2230,
- REICH, H. J.; RIGBY, J. H.: "Handbook of Reagents for Organic Synthesis: Acidic and Basic Reagents" 1999, JOHN WILEY & SONS , CHICHESTER , XP002357298 Pages 300-303: Potassium Fluoride.

## Description

The invention relates to a process for the preparation of 5-methylamino-1-phenylpyrazole intermediates which can be used to prepare 5-alkylthioethylamino-1-phenyl-pyrazoles.
5-Alkylthioethylamino-1-phenyl-pyrazoles are described as ectoparasiticides and crop insecticides in WO 03/074493.

These compounds can be prepared from 5-aminopyrazoles or 5-methylaminopyrazoles as described in WO 03/074493. The disadvantage of the methods described are the low yields and the difficult and expensive purification work required for the preparation of high purity samples

A reliable and simple process has surprisingly now been found for the preparation of some 5-alkylthioalkylamino-1-phenyl-pyrazoles in accordance to formula (I), that provides high yields of products with high purity and decreases side products and thereby facilitates purification of large quantities.
5-Alkylthioethylamino-1-phenyl-pyrazoles (I), 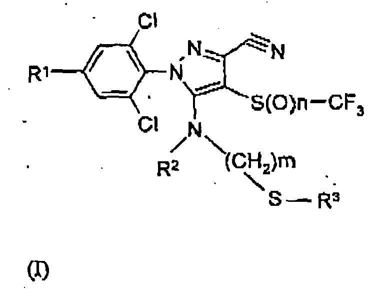 in which
- R¹: is (C₁-C₃)-haloalkyl or (C₁-C₃)-haloalkoxy
- R²: is H or (C₁-C₆)-alkyl
- R³: is (C₁-C₆)-alkyl
and n is 0, 1 or 2 and m is 1, 2 or 3,
preferably R¹ is CF₃ or OCF₃, R² is methyl, ethyl or propyl and R³ is methyl, ethyl or propyl and m is 2,
can be prepared by a process in which a 5-amino-1-phenyl-pyrazole (II), 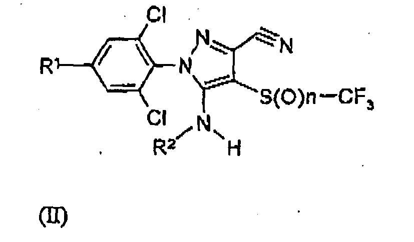 where R¹, R² and n are as defined above,
is reacted with an alkylthioalkylchloride (III)
R³-S-(CH₂)ₘ-Cl (III)
preferably with 2-alkylthioethylchloride (R³-S-CH₂-CH₂-Cl), in which R³ and m are as defined above,
in the presence of a base comprising a basic potassium salt or a mixture of potassium salts, preferably in the presence of a basic potassium salt, e.g. selected from the group consisting of potassiumcarbonate, potassiumfluoride, tripotassiumphosphate or a mixture thereof, and in a solvent comprising a nitrile or a mixture of nitriles, preferably in a nitrile. e.g. selected from the group consisting of acetonitrile, propionitrile or a mixture thereof, preferably at a temperature between 20-100°C, preferably within a period of 1- 8 hours.

The used base comprise at least one basic potassium salt preferably in an amount of at least 25 weight-%, more preferred in an amount of at least 75 weight-%, in particular preferred of at least 90 weight-% (the weight-% amounts are related to the total base amount). The used solvent comprise at least one nitrile preferably in an amount of at least 50 volume%, more preferred in an amount of at least 75 volume%, in particular preferred in an amount of at least 90 volume-% (the volume-% amount is related to the total amount of solvent).

The starting materials for the claimed method are well known. Phenylpyrazole compounds of formula (II) wherein R² is alkyl are e.g. known from US 6,531,501 B1. 1-Phenyl-5-alkylaminopyzoles synthesis methods are known from US 6,531,501 B1, DE 3719732 and GB 2123420. Compounds of formula (II) wherein R² is H are known from EP 295117.

In the prior art process (WO 03/074493) the 5-alkylthioalkylamino-1-phenyl-pyrazoles of formula (I) are prepared with several starting material, with bases like sodium hydride in solvents like tetrahydrofuran and, dimethylformamide (DMF) or dioxane. The yields are quite low, from 10% to 39% (see table 5). Extensive purification work is than required to isolate the products with >95% purity.

A particular technical and economic advantage of the new process compared to the known synthesis is that yields above 70% were achieved, sometimes even above 80 % (table 5). The improvement can be surprisingly achieved by a preferred combination of specific starting materials and in particular an advantageous combination of an appropriate base and solvent.

The number and quantity of side products are decreased tremendously and it is possible to purify the compounds of formula (I) to >95% purity by column chromatography or by recrystallisation. No HPLC separation is necessary to achieve purity above 95%, The preparation of samples from 10-100 g and more, with purity >95%, become economically feasible by using the described method.

The invention relates to a process for the preparation of compounds of the formula (IIa) 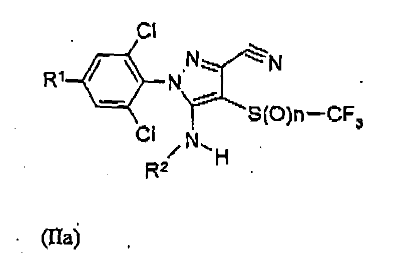 in which
- R¹: is (C₁-C₃)-haloalkyl or (C₁-C₃-haloalkoxy.
- R²: is (C₁-C₆)-alkyl
and n is 0, 1 or 2,
preferably R¹ is CF₃ or OCF₃, R² is methyl, ethyl or propyl -
from compounds of formula (IIb) by application of a 3-step reaction sequence.

Compounds of formula (IIb) are known from EP 0 295 117.

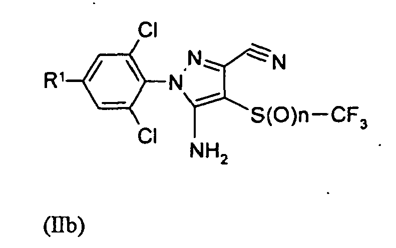

The known preparation methods for compounds of formula (IIa) (described e.g. in US 6,531,501 B1, DE 3719732 and GB 2123420) suffer from low yields, side reactions, reproducibility problems, insufficient purity and difficult purification steps within the processes.

A reliable and simple process has surprisingly now been found for the preparation of 5-allcylamino-1-phenyl-pyrazoles (I), that provides high yields of products with high purity and decreases side products and thereby facilitates purification of large quantities.

The process for the preparation of compounds of the formula (IIa) wherein R² is (C₁-C₆)-alkyl from formula (IIb) wherein R² is H involves three reaction steps:

### 1. Step: Acylation of the aminopyrazole (IIb) with alkanecarboxylic anhydride, e.g. an compound of the formula (((C₁-C₆)-Alkyl)CO)₂O, in particular acetanhydride.

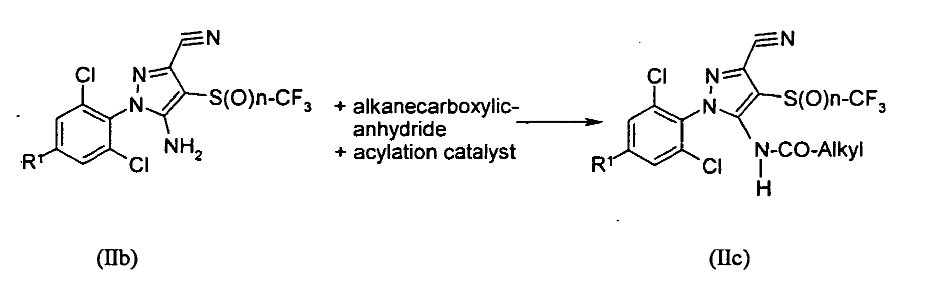

This type of reaction is already known from DE 3719732 (Bayer AG).

It is applied to formula (IIb) with good success.

The reaction is performed in organic solvents e.g. like THF, dioxane or toluene in the presence of acylation catalysts e.g. N-containing heteroaromatic compounds, preferably pyrimidine, pyridazine, pyrazine, triazine or pyridine or derivatives thereof, like dimethylaminopyridine (DMAP) preferably at temperatures from 50 - 120°C. High yields and good purity is achieved, optionally known purification procedures may be used in addition.

### 2. Step: Alkylation of the amide group at the N-atom

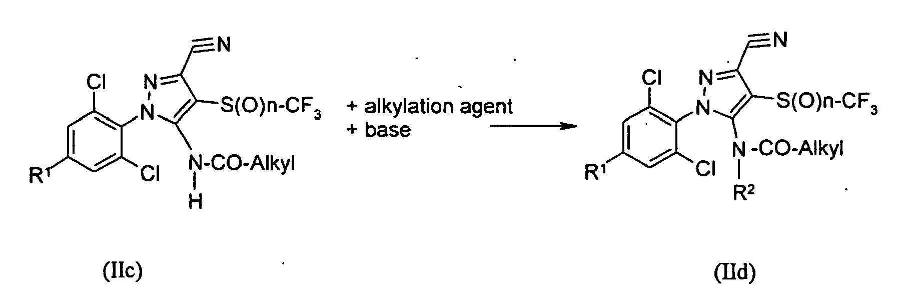

The reaction is performed in organic solvents e.g. like DMF, DMSO (dimethylsulfoxide), acetonitrile in the presence of alkylation agents, preferably alkylhalogenides e.g. like alkyliodides, alkylbromides and bases, preferably basic alkali salts e.g. such as alkalicarbonates, alkalihydrides, alkaliphosphates, alkalihydroxides preferably at temperatures from 20-100°C. High yields and good purity is achieved, optionally known purification procedures may be used in addition.

### 3. Step: Acidic hydrolysis of the N-alkylamid group of compound (IId) to yield compound (IIa)

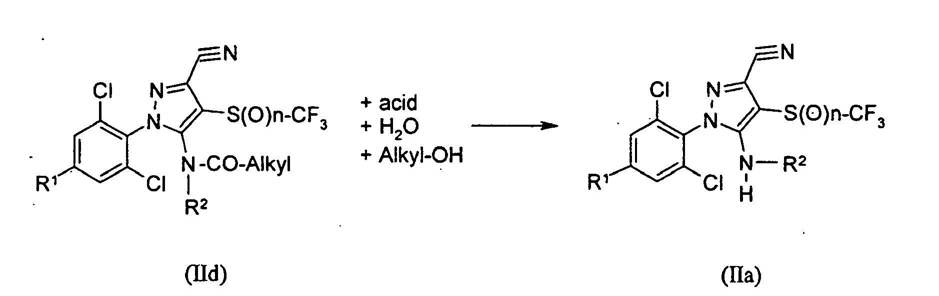

The reaction is performed with a protonic acid, in particular sulfuric acid and water in organic solvents e.g. like (C₁-C₅)-alkanols, preferred methanol, ethanol, propanol, butanol and pentanol of the formula CₙH₂ₙ₊₁OH (n = 1-5), preferably at temperatures from 60 - 130°C. The acid strength of the acid is adjusted by mixing it with an alcohol and water according to the requirements of the reacting compounds. Preferred are water : alcohol mixtures (v/v) from 1 : 0,5 to 1 : 50, more preferred from 1 : I to 1 : 20, in particular from 1 : 2 to 1 : 10. The protonic acid may be added carefully to the reaction mixture, preferably 1,5 mol to 10 mol, in particular between 2 mol and 6 mol acid in relation to 1 mol starting material (IId). High yields and good purity is achieved optionally known purification procedures may be used in addition.

It was not possible to perform the amide hydrolysis under basic conditions or under other acidic conditions due to the sensitivity of the groups 4-S(O)ₙCF₃ and 3-CN on the pyrazole. Those groups are attacked by nucleophiles or by very strong acids before the amide hydrolysis can take place. Therefore the hydrolysis method described in DE 3719732 is not applicable to compounds (IId). A complex mixture of products was obtained with less than 10 % of compound (IIa).

A particular economic and chemical advantage compared with the known methods is that overall yields above 55-65 % are achieved. All product compounds can be purified by recrystallisation. Batches of formula (IIa) are obtained with purities >97% . The content of formula (IIb) in purified batches of formula (IIa) is below 0.5 % . The preparation of high quality batches from 100 g to 1 kg or more are easy possible and become economically feasible using the described method.

The resulting compounds in accordance to formula (IIa) may be used as starting compounds in the above described method for the preparation of 5-alkylthioalkylamino-1-phenyl-pyrazoles of formula (I).

In addition suitable pesticidally acceptable salts of 5-alkylthioalkylamino-1-phenyl-pyrazoles of formula (I) may be prepared with acids. By the term "pesticidally acceptable salts" is meant salts the anions or cations of which are known and accepted in the art for the formation of salts for pesticidal use. Suitable salts with acids, e.g. formed by compounds of formula (I) with inorganic acids, for example hydrochlorides, sulphates, phosphates and nitrates and salts with organic acids for example acetic acid or (methane)sulfonic acid.

In the present specification, including the accompanying claims, the aforementioned substituents have the following meanings:
"Alkyl" -groups and portions thereof may be straight- or branched-chain.

The expression "(C₁-C₆)-alkyl" is to be understood as meaning an unbranched or branched hydrocarbon radical having 1, 2, 3, 4, 5 or 6 carbon atoms, such as, for example a methyl, ethyl, propyl, isopropyl, 1-butyl, 2-butyl, 2-methylpropyl or tert.-butyl radical. Alkyl radicals and also in composite groups, unless otherwise defined, preferably have 1 to 3 carbon atoms.

The term "halo" before the name of a radical means that this radical is partially or completely halogenated, that is to say, substituted by F, Cl, Br, or I, in any combination, preferably by F or Cl.

"(C₁-C₆)-haloalkyl" means an alkyl group mentioned under the expression "(C₁-C₆)-alkyl" in which one or more hydrogen atoms are replaced by the same number of identical or different halogen atoms, such as monohaloalkyl, perhaloalkyl, CF₃, CHF₂, CH₂F, CHFCH₃, CF₃CH₂, CF₃CF₂, CHF₂CF₂, CH₂FCHCl, CH₂Cl, CCl₃, CHCl₂ or CH₂CH₂Cl.

"(C₁-C₆)-alkoxy" means an alkoxy group whose carbon chain has the meaning given under the expression "(C₁-C₆)-alkyl". "Haloalkoxy" is, for example, OCF₃, OCHF₂, OCH₂F, CF₃CF₂O, OCH₂CF₃ or OCH₂CH₂Cl.

Preferred chemical examples of compounds of formula (I) and (II) which are or may be prepared with the described method are listed in tables 1 to 4.

Where subscripts are omitted in the tables they are intended, for example CF3 means CF₃. In the Tables Me means methyl, Et means ethyl, Pr means propyl, Bu means butyl. Compound numbers are given for reference purposes only.

**Table 1:**

| R¹ is CF₃; variation of R² and R³, n | | | | | |
|---|---|---|---|---|---|
| Compound number | | R² | R³ | n | NMR ¹H or ¹⁹F (ppm) |
| 01- | 01 | H | Me | 0 | |
| 01- | 02 | H | Me | 1 | |
| 01- | 03 | H | Me | 2 | |
| 01- | 04 | H | Et | 0 | |
| 01- | 05 | H | Et | 1 | |
| 01- | 06 | H | Et | 2 | |
| 01- | 07 | H | nPr | 0 | |
| 01- | 08 | H | nPr | 1 | |
| 01- | 09 | H | nPr | 2 | |
| 01- | 10 | H | iPr | 0 | |
| 01- | 11 | H | iPr | 1 | |
| 01- | 12 | H | iPr | 2 | |
| 01- | 13 | Me | Me | 0 | ¹⁹F: -44,66; -64,13; |
| 01- | 14 | Me | Me | 1 | ¹⁹F: -64,17; -72,89; |
| 01- | 15 | Me | Me | 2 | ¹H: 2,00; 2,49; 2,91; 3,16; 7,84; |
| 01- | 16 | Me | Et | 0 | |
| 01- | 17 | Me | Et | 1 | |
| 01- | 18 | Me | Et | 2 | |
| 01- | 19 | Me | nPr | 0 | |
| 01- | 20 | Me | nPr | 1 | |
| 01- | 21 | Me | nPr | 2 | |
| 01- | 22 | Me | iPr | 0 | |
| 01- | 23 | Me | iPr | 1 | |
| 01- | 24 | Me | iPr | 2 | |
| 01- | 25 | Et | Me | 0 | |
| 01- | 26 | Et | Me | 1 | |
| 01- | 27 | Et | Me | 2 | |
| 01- | 28 | Et | Et | 0 | |
| 01- | 29 | Et | Et | 1 | |
| 01- | 30 | Et | Et | 2 | |
| 01- | 31 | Et | nPr | 0 | |
| 01- | 32 | Et | nPr | 1 | |
| 01- | 33 | Et | nPr | 2 | |
| 01- | 34 | Et - | iPr | 0 | |
| 01- | 35 | Et | iPr | 1 | |
| 01- | 36 | Et | iPr | 2 | |
| 01- | 37 | nPr | Me | 0 | |
| 01- | 38 | nPr | Me | 1 | |
| 01- | 39 | nPr | Me | 2 | |
| 01- | 40 | nPr | Et | 0 | |
| 01- | 41 | nPr | Et | 1 | |
| 01- | 42 | nPr | Et | 2 | |
| 01- | 43 | nPr | nPr | 0 | |
| 01- | 44 | nPr | nPr | 1 | |
| 01- | 45 | nPr | nPr | 2 | |
| 01- | 46 | nPr | iPr | 0 | |
| 01- | 47 | nPr | iPr | 1 | |
| 01- | 48 | nPr | iPr | 2 | |
| 01- | 49 | nPr | Me | 0 | |
| 01- | 50 | iPr | Me | 1 | |
| 01- | 51 | iPr | Me | 2 | |
| 01- | 52 | iPr | Et | 0 | |
| 01- | 53 | iPr | Et | 1 | |
| 01- | 54 | iPr | Et | 2 | |
| 01- | 55 | iPr | nPr | 0 | |
| 01- | 56 | iPr | nPr | 1 | |
| 01- | 57 | iPr | nPr | 2 | |
| 01- | 58 | iPr | iPr | 0 | |
| 01- | 59 | iPr | iPr | 1 | |
| 01- | 60 | iPr | iPr | 2 | |

**Table 2 :**

| R² is Me; variation of R¹, R³, n | | | | | |
|---|---|---|---|---|---|
| Compound number | | R¹ | R³ | n | NMR ¹H or ¹⁹F (ppm) |
| 02- | 01 | OCF3 | Me | 0 | |
| 02- | 02 | OCF3 | Me | 1 | |
| 02- | 03 | OCF3 | Me | 2 | |
| 02- | 04 | OCF3 | Et | 0 | |
| 02- | 05 | OCF3 | Et | 1 | |
| 02- | 06 | OCF3 | Et | 2 | |
| 02- | 07 | OCF3 | nPr | 0 | |
| 02- | 08 | OCF3 | nPr | 1 | |
| 02- | 09 | OCF3 | nPr | 2 | |
| 02- | 10 | OCF3 | iPr | 0 | |
| 02- | 11 | OCF3 | iPr | 1 | |
| 02- | 12 | OCF3 | iPr | 2 | |

**Table 3:**

| Pyrazolamides of formula (IIc) and (IId) | | | | | |
|---|---|---|---|---|---|
| Compound number | | R¹ | R² | n | NMR ¹H or ¹⁹F (ppm) |
| 03- | 01 | CF3 | H | 0 | |
| 03- | 02 | CF3 | H | 1 | |
| 03- | 03 | CF3 | H | 2 | |
| 03- | 04 | OCF3 | H | 0 | |
| 03- | 05 | OCF3 | H | 1 | |
| 03- | 06 | OCF3 | H | 2 | |
| 03- | 07 | CF3 | Me | 0 | |
| 03- | 08 | CF3 | Me | 1 | |
| 03- | 09 | CF3 | Me | 2 | |
| 03- | 10 | CF3 | Et | 0 | |
| 03- | 11 | CF3 | Et | 1 | |
| 03- | 12 | CF3 | Et | 2 | |
| 03- | 13 | CF3 | nPr | 0 | |
| 03- | 14 | CF3 | nPr | 1 | |
| 03- | 15 | CF3 | nPr | 2 | |
| 03- | 16 | CF3 | iPr | 0 | |
| 03- | 17 | CF3 | iPr | 1 | |
| 03- | 18 | CF3 | iPr | 2 | |
| 03- | 19 | OCF3 | Me | 0 | |
| 03- | 20 | OCF3 | Me | 1 | |
| 03- | 21 | OCF3 | Me | 2 | |
| 03- | 22 | OCF3 | Et | 0 | |
| 03- | 23 | OCF3 | Et | 1 | |
| 03- | 24 | OCF3 | Et | 2 | |
| 03- | 25 | OCF3 | nPr | 0 | |
| 03- | 26 | OCF3 | nPr | 1 | |
| 03- | 27 | OCF3 | nPr | 2 | |
| 03- | 28 | OCF3 | iPr | 0 | |
| 03- | 29 | OCF3 | iPr | 1 | |
| 03- | 30 | OCF3 | iPr | 2 | |

**Table 4**

| 5-Alkylaminopyrazoles of formula (IIa) | | | | | |
|---|---|---|---|---|---|
| Compound number | | R¹ | R² | n | NMR ¹H or ¹⁹F (ppm) |
| 04- | 01 | CF3 | Me | 0 | |
| 04- | 02 | CF3 | Me | 1 | |
| 04- | 03 | CF3 | Me | 2 | |
| 04- | 04 | CF3 | Et | 0 | |
| 04- | 05 | CF3 | Et | 1 | |
| 04- | 06 | CF3 | Et | 2 | |
| 04- | 07 | CF3 | nPr | 0 | |
| 04- | 08 | CF3 | nPr | 1 | |
| 04- | 09 | CF3 | nPr | 2 | |
| 04- | 10 | CF3 | iPr | 0 | |
| 04- | 11 | CF3 | iPr | 1 | |
| 04- | 12 | CF3 | iPr | 2 | |
| 04- | 13 | OCF3 | Me | 0 | |
| 04- | 14 | OCF3 | Me | 1 | |
| 04- | 15 | OCF3 | Me | 2 | |
| 04- | 16 | OCF3 | Et | 0 | |
| 04- | 17 | OCF3 | Et | 1 | |
| 04- | 18 | OCF3 | Et | 2 | |
| 04- | 19 | OCF3 | nPr | 0 | |
| 04- | 20 | OCF3 | nPr | 1 | |
| 04- | 21 | OCF3 | nPr | 2 | |
| 04- | 22 | OCF3 | iPr | 0 | |
| 04- | 23 | OCF3 | iPr | 1 | |
| 04- | 24 | OCF3 | iPr | 2 | |

### Synthetic Examples

NMR spectra were run in deuterochloroform unless stated otherwise, and shifts are given in ppm.

In the examples which follow, quantities (also percentages) are weight based, unless stated otherwise.

The following non-limiting examples illustrate the preparation of the compounds of formula (I).

1-(2,6-Dichloro-4-trifluoromethylphenyl)-3-cyano-5-(N-methyl-N-(2-methylthioethyl)amino)-4-trifluoromethylthiopyrazole (compound no. 01-01)

To a mixture of 1-(2,6-dichloro-4-trifluoromethylphenyl)-3-cyano-5-amino-4-trifluoromethyl-thiopyrazole (3.00 g, 6.9 mmol) and potassium carbonate (2.85 g, 20.6 mmol) in acetonitrile (45 ml) was added 2-chloroethyl-methylsulfide (0.84 g, 7.5 mmol) at 20-30°C. The mixture was heated to reflux for 70 minutes. Extractive workup (heptane-ethyl acetate, water) and recrystallisation from heptane-ethylacetate gave the title product (Compound 01-01, 1.33 g) as a solid.

purity >98%; 1H-NMR: 2,01, SMe; 2,64, CH2S; 3,49, NCH2; 4,72, NH; 7,80, ArH;

1-(2,6-Dichloro-4-trifluoromethylphenyl)-3-cyano-5-(N-methyl-N-(2-methylthioethyl)amino)-4-trifluoromethylthiopyrazole (compound no. 01-13)

To a mixture of 1-(2,6-dichloro-4-trifluoromethylphenyl)-3-cyano-5-methylamino-4-trifluoromethylthiopyrazole (2.00 g, 4.6 mmol) and tripotassium phosphate (2.93 g, 13.8 mmol) in acetonitrile (40 ml) was added 2-chloroethyl-methylsulfide (0.61 g, 5.5 mmol) at 20-30°C. The mixture was heated to reflux for 8 hours. Extractive workup (heptane-ethyl acetate, water) and column chromatography with heptane-ethylacetate gave the title product (Compound 01-13, 1.76 g) as a solid.

purity 94%; 1H-NMR: 1,99, SMe; 2,44, CH2S; 2,92, NMe; 3,23, NCH2; 7,79, ArH;

After recrystallisation purity >97% can be achieved.

1-(2,6-Dichloro-4-trifluoromethylphenyl)-3-cyano-5-(N-methyl-N-(2-methylthioethyl)amino)-4-trifluoromethylsulfinylpyrazole (compound no. 01-14)

To a mixture of 1-(2,6-dichloro-4-trifluoromethylphenyl)-3-cyano-5-methylamino-4-trifluoromethylsulfinylpyrazole (3.00 g, 6.65 mmol) and potassium carbonate (2.76 g, 20.0 mmol) in acetonitrile (40 ml) was added 2-chloroethyl-methylsulfide (0.80 g, 7.3 mmol) at 20-30°C. The mixture was heated to reflux for 4 hours. Extractive workup (heptane-ethyl acetate, water) and column chromatography with heptane-ethylacetate gave the title product (Compound 01-14, 2.31 g) as a solid.

purity 93%; 1 H-NMR: 2,00, SMe; 2,48, CH2S; 2,89, NMe; 3,13, NCH2; 7,82, ArH;

After recrystallisation purity >97% can be achieved.

**Table 5:**

| Comparison of Yields of formula (I) compounds achieved by different methods | | | | |
|---|---|---|---|---|
| Comp. No. | Base | Solvent | Yield (NMR) | Yield isolated |
| 01-13 | K3PO4 | acetonitrile | 89% | 79% |
| 01-13 | K3PO4 | NMP | 7% | |
| 01-13 | NaH | DMF | 10 % | |
| 01-13 | NaH | DMF-DMSO | 10 % | |
| | | | | |
| 01-14 | K2CO3 | acetonitrile | 85% | 82 % |
| 01-14 | K3PO4 | acetonitrile | 89% | |
| 01-14 | K3PO4 | dioxane | 12 % | |
| 01-14 | K3PO4 | DMF-DMSO | 26 % | |
| 01-14 | K3PO4 | dichloroethane | 0 % | |
| 01-14 | NaH | DMF | 39% | |
| 01-14 | NaH | acetonitrile | 58 % | |
| 01-14 | Na2CO3 | acetonitrile | 13 % | |

The following non-limiting examples illustrate the preparation of the compounds of formula (IIc), (IId) and (IIa).

1-(2,6-Dichloro-4-trifluoromethylphenyl)-3-cyano-5-acetylamino-4-trifluoromethylsulfinylpyrazole (compound no. 03-02)

To a mixture of 1-(2,6-dichloro-4-trifluoromethylphenyl)-3-cyano-5-amino-4-trifluoromethyl-sulfinylpyrazole (90.0 g, 206 mmol), pyridine (3.2 g, 41.2 mmol) and 4-dimethylaminopyridine (1.05 g, 10 mmol) in tetrahydrofuran (600 ml) was added acetic anhydride (42.0 g, 412 mmol) at 20-30°C. The mixture was heated to reflux for 7 hours. Extractive workup (heptane-ethyl acetate, water) and recrystallisation from ethanol gave the title product (Compound 03-02, 81.3 g) as a solid.

purity >98%; ¹⁹F-NMR: -63,7 (Ph-CF₃); -73,8 (SOCF₃);

### 1-(2,6-Dichloro-4-trifluoromethylphenyl)-3-cyano-5-N-acetyl-N-methylamino-4-trifluoromethylsulfinylpyrazole (compound no. 03-08)

To a mixture of 1-(2,6-dichloro-4-trifluoromethylphenyl)-3-cyano-5-acetylamino-4-trifluoromethyl-sulfinylpyrazole (50.0 g, 105 mmol) and potassium carbonate (17.6 g, 126 mmol) in DMF (150 ml) was added methyliodide (21.1 g, 147 mmol) at 20-30°C. The mixture was heated to 35-40°C for 8 hours. Extractive workup (heptane-ethyl acetate, water) and recrystallisation from ethanol gave the title product (Compound 03-08, 43.0 g) as a solid; purity >97%; ¹⁹F-NMR: -63,8 (Ph-CF₃); -72,2-72,5 (SO-CF₃).

### 1-(2,6-Dichloro-4-trifluoromethylphenyl)-3-cyano-5-methylamino-4-trifluoromethylsulfinylpyrazole (compound no. 04-02)

To a mixture of 1-(2,6-dichloro-4-trifluoromethylphenyl)-3-cyano-5-N-methyl-N-acetylamino-4-trifluoromethylsulfinylpyrazole (50.0 g, 100 mmol) and water (100 ml) in n-butanol (400 mL) was added carefully concentrated sulfuric acid (39.6 g, 400 mmol) at 20-50°C. The mixture was heated to reflux for 18 hours. The reaction mixture is diluted with water and the precipitate is isolated by filtration. Recrystallisation from ethanol gave the title product (Compound 04-02, 40.0 g) as a solid. purity >98%; ¹H-NMR: 2,61 (NMe); 5,93 (NH); 7,80 (ArH).

## Claims

1. A process for the preparation of compounds of formula (IIa) 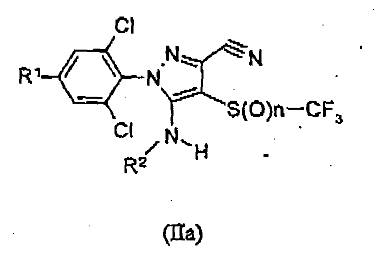 in which
R¹ is (C₁-C₃)-haloalkyl or (C₁-C₃)-haloalkoxy
R² is (C₁-C₆)-alkyl
and n is 0, 1 or 2,
from compounds of formula (IIb) 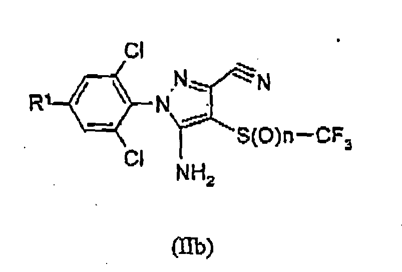 comprising
a) the acylation of the aminopyrazole (IIb) with an alkanecarboxylic anhydride, 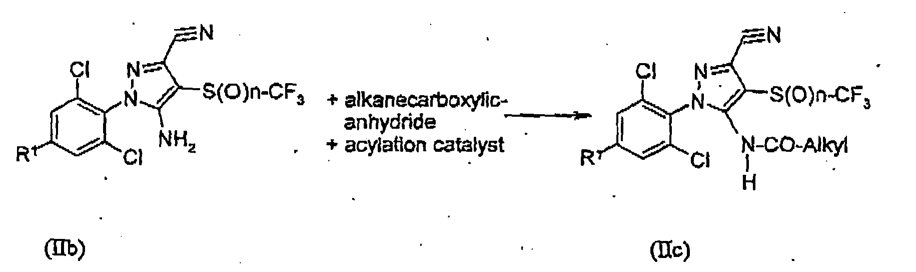 in organic solvents and in the presence of acylation catalysts,
b) alkylation of the amide group at the N-atom 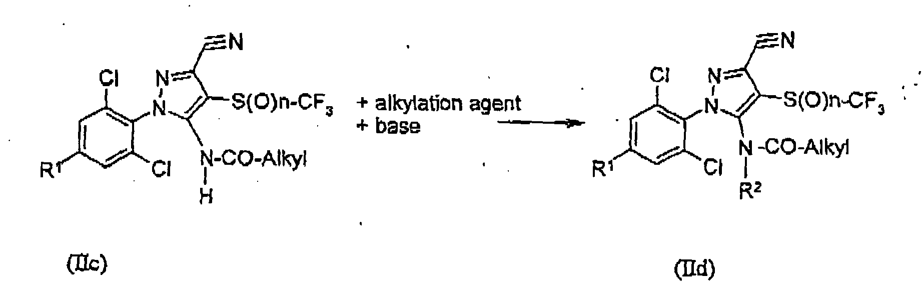 in an organic solvent and in the presence of alkylation agents and a base, and
c) acidic hydrolysis of the N-alkylamid group of compound (IId) to yield compound (IIa) 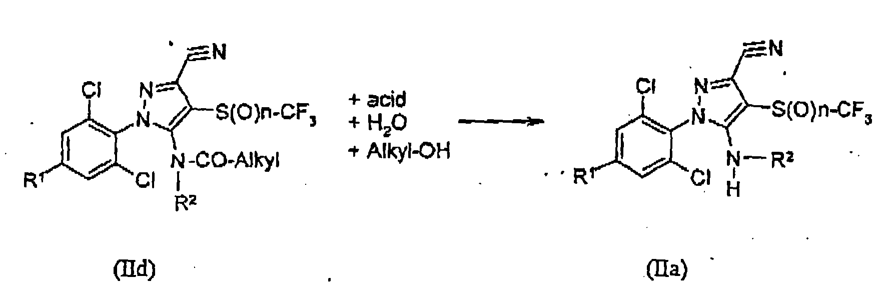 with a protonic acid and water in organic solvents and at least one alcohol,

2. A process as claimed in claim 1, wherein compounds of formula (II) in which R¹ is CF₃ or OCF₃ and/or R² is methyl, ethyl or propyl are used.

3. A process as claimed in claim 1 or 2, wherein the reaction step c) is carried out in the presence of sulfuric acid.

4. A process as claimed in any one of claims 1 to 3, wherein the organic solvent and/or the alcohol is selected from (C₁-C₅)-alkanols.

## Patentansprüche

1. Verfahren zur Herstellung von Verbindungen der Formel (IIa) 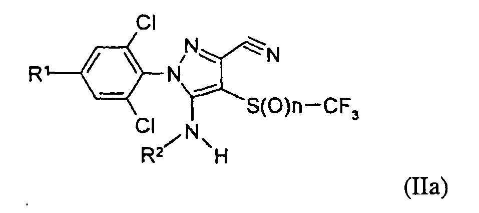 wobei
R¹ die Bedeutung (C₁-C₃)-Halogenalkyl oder (C₁-C₃)-Halogenalkoxy hat,
R² die Bedeutung (C₁-C₆)-Alkyl hat und
n gleich 0, 1 oder 2 ist,
ausgehend von Verbindungen der Formel (IIb) 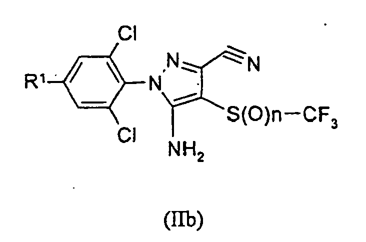 umfassend
a) die Acylierung des Aminopyrazols (IIb) mit einem Alkancarbonsäureanhydrid 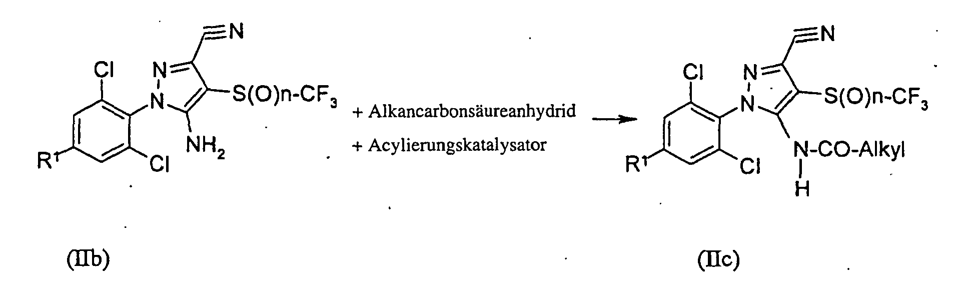 in organischen Lösungsmitteln und in Gegenwart von Acylierungskatalysatoren,
b) Alkylierung der Amidgruppe am Stickstoffatom 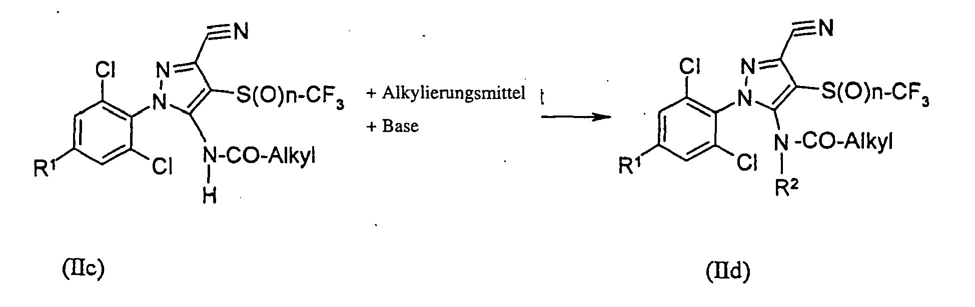 in einem organischen Lösungsmittel und in Gegenwart von Alkylierungsmitteln und einer Base, und
c) saure Hydrolyse der N-Alkylamidgruppe der Verbindung (IId), um Verbindung (IIa) zu gewinnen, 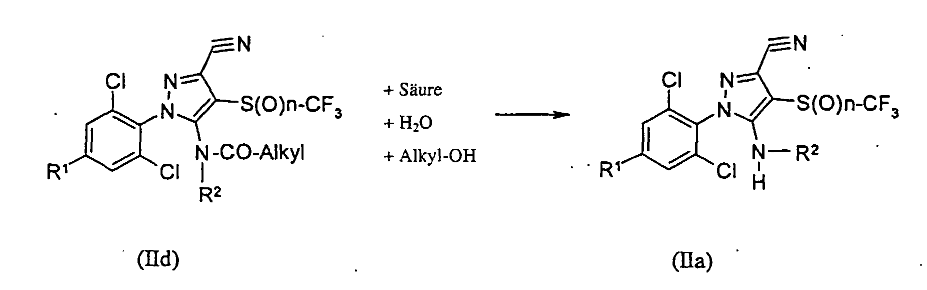 mit einer Brönsted-Säure und Wasser in organischen Lösungsmitteln und mindestens einem Alkohol.

2. Verfahren gemäß Anspruch 1, wobei Verbindungen der Formel (II) verwendet werden, in welchen R¹ die Bedeutung CF₃ oder OCF₃ hat und/oder R² Methyl, Ethyl oder Propyl ist.

3. Verfahren gemäß Anspruch 1 oder 2, wobei der Reaktionsschritt c) in Gegenwart von Schwefelsäure durchgeführt wird.

4. Verfahren gemäß einem der Ansprüche 1 bis 3, wobei das organische Lösungsmittel und/oder der Alkohol aus (C₁-C₅)-Alkanolen ausgewählt ist.

## Revendications

1. Procédé pour la préparation de composés de formule (IIa) 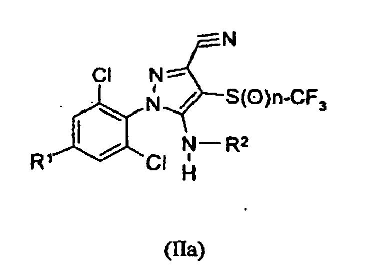 où
R¹ est (C₁-C₃)-haloalkyle ou (C₁-C₃)-haloalcoxy
R² est (C₁-C₆)-alkyle
et n est 0, 1 ou 2,
à partir de composés de formule (IIb) 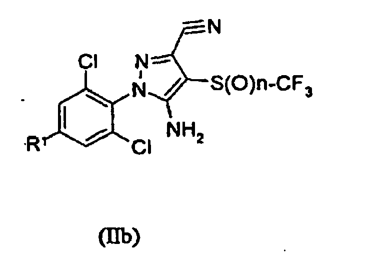 comprenant
a) l' acylation de l'aminopyrazole (IIb) avec un anhydride alcanecarboxylique, 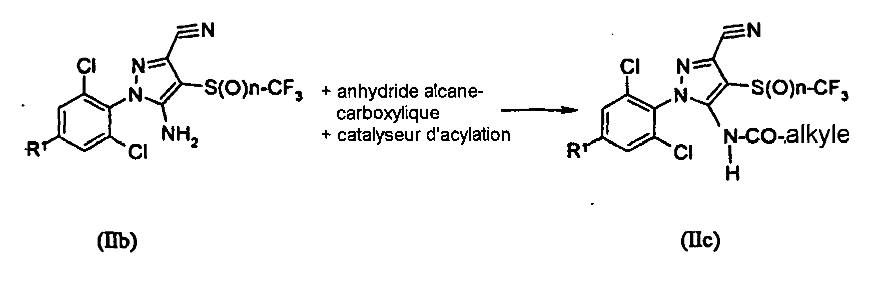 dans des solvants organiques et en présence de catalyseurs d'acylation,
b) l'alkylation du groupe amide sur l'atome N 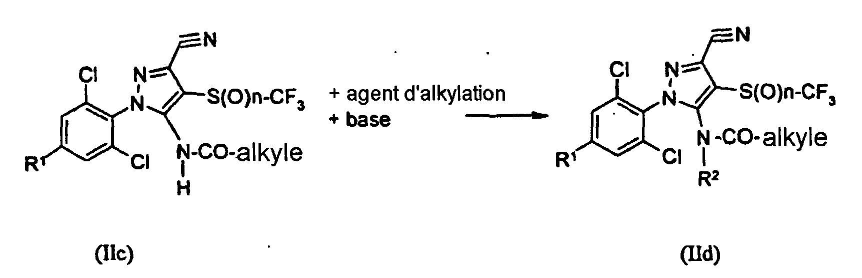 dans un solvant organique et en présence d'agents d'alkylation et d'une base, et
c) l'hydrolyse acide du groupe N-alkylamide du composé (IId) pour former le composé (IIa) 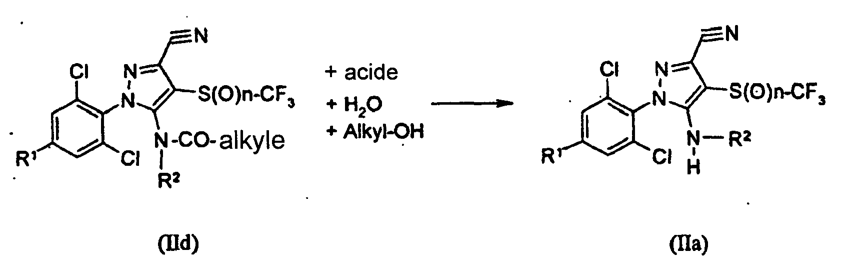 avec un acide protonique et de l'eau dans des solvants organiques et au moins un alcool.

2. Procédé selon la revendication 1 où des composés de formule (II) où R¹ est CF₃ ou OCF₃ et/ou R² est méthyle, éthyle ou propyle sont utilisés.

3. Procédé selon la revendication 1 ou 2 où l'étape réactionnelle c) est conduite en présence d'acide sulfurique.

4. Procédé selon l'une quelconque des revendications 1 à 3 où le solvant organique et/ou l'alcool sont choisis parmi les (C₁-C₅)-alcanols.
